# EUROPEAN PATENT APPLICATION

(11) **EP 3 517 674 A1**
(43) Date of publication of application: **31.07.2019**
(21) Application number: 18153845.5
(22) Date of filing: 29.01.2018
(51) Int. Cl.: D04H 1/54, D04H 1/555, A61F 13/511, A61F 13/533

(54) **APPARATUS AND METHOD FOR ULTRASONIC BONDING**

(71) Applicant: The Procter & Gamble Company, Cincinnati, OH 45202 (US)
(72) Inventor: DIAS, Marcelo, 53881 Euskirchen (DE); ENDRES, Joerg, 65824 Schwalbach (DE); GARCIA, Julien René, 65824 Schwalbach am Taunus (DE); GUPTA, Sudhanshu, 53881 Euskirchen (DE); MISHRA, Piyush, 53881 Euskirchen (DE); NUNEZ, Luis Eduardo, 35881 Euskirchen (DE)
(74) Representative: Mather, Peter Geoffrey

(57) **Abstract**

The present invention relates to a method for ultrasonic bonding and to an apparatus comprising an ultrasonic horn and an anvil roll,
wherein the anvil roll comprises a plurality of raised pattern elements around the circumference of the anvil roll,
wherein the pattern elements comprising a pattern surface comprising a plurality of pins, the raised pattern surface has a varying width profile, in the cross-machine direction, around the circumference of the anvil roll,
wherein at a first circumferential position, a first part of the pattern surface, having a first width, interacts with the ultrasonic horn to provide a maximum bonding density, Wmax, and
wherein at a second circumferential position, a second part of the pattern surface, having a second width, interacts with the ultrasonic horn to provide a minimum bonding density, Wmin,
wherein the first and second parts of the pattern surface are configured so that the ratio of ultrasonic energy transfer at the first position to the ultrasonic energy transfer at the second position is from 1.1:1 to 10:1., preferably from 1.5:1 to 3:1.

## Description

### Field of the Invention

The present invention relates to apparatus and method for ultrasonic bonding. In particular the apparatus and method may provide a pattern surface of bond areas, for example bonding together supporting sheets of an absorbent core used in disposable hygiene products.

### Background of the Invention

It is known in the art that nonwoven substrates or webs can be bonded together by ultrasonic means. US 5,817,199, published on October 6th 1998, discloses an ultrasonic bonding device comprising an anvil roll receiving web materials thereon and receiving at least two rotary ultrasonic horns. The rotary ultrasonic horns, in combination with the anvil roll, ultrasonically bond segments of e.g. first and second webs to each other.

Whereas US 5,817,199 teaches a method to provide ultrasonic bonding across the full width of the consolidated webs, it may be desirable to provide different widths of ultrasonic bonding at different positions along the machine direction of the consolidated webs. For example, in continuous manufacturing processes which are designed to produce absorbent cores for disposable hygiene articles, the continuous web may be ultrasonically bonded prior to being cut into discrete lengths which correspond to individual absorbent cores. In such a process it may be desirable to provide ultrasonic bonding across the full width of the consolidated webs in the end region of the individual absorbent cores, and to provide relatively narrow ultrasonic bonding, e.g. in the product center or alongside margins which are spaced away from the end regions along the product length or machine direction, in such a way that the absorbent core is sealed and absorbent material is prevented from escaping from within the absorbent core.

The method described in the prior art, including in US 5,817,199, may use multiple ultrasonic horns in order to achieve an ultrasonic bond of the desired width. However multiple ultrasonic horns are expensive to procure, install, maintain and operate.

It would be more economical to use one anvil roll in combination with one ultrasonic horn. However in this configuration the problem remains that the ultrasonic horn and the anvil roll need to be configured so that a comparatively large amount of ultrasonic energy is transmitted to the bonding area in the widest region of the bonding area (e.g. the end regions), whereas a comparatively small amount of ultrasonic energy is transmitted to the bonding area in the narrowest region of the bonding area (e.g. center of the product or/and the side margins).

The present invention provides methods and apparatuses with which to address the problems set out above.

### Summary of the Invention

In one aspect the present invention relates to an apparatus comprising an ultrasonic horn and an anvil roll,
wherein the anvil roll comprises a plurality of raised pattern elements around the circumference of the anvil roll,
wherein the pattern elements comprising a pattern surface comprising a plurality of pins, the raised pattern surface has a varying width profile, in the cross-machine direction, around the circumference of the anvil roll,
wherein at a first circumferential position, a first part of the pattern surface, having a first width, interacts with the ultrasonic horn to provide a maximum bonding density, Wmax, and
wherein at a second circumferential position, a second part of the pattern surface, having a second width, interacts with the ultrasonic horn to provide a minimum bonding density, Wmin,
wherein the first and second parts of the pattern surface are configured so that the ratio of ultrasonic energy transfer at the first position to the ultrasonic energy transfer at the second position is from 1.1:1 to 10:1., preferably from 1.5:1 to 3:1.

In a second aspect the present invention relates to a method comprising the steps of:
advancing a composite of at least first and second supporting sheet through a bonding region, wherein the bonding region comprises an ultrasonic horn and an anvil roll, the anvil roll comprising a plurality of raised pattern elements around the circumference of the anvil roll, the pattern elements comprising a pattern surface comprising a plurality of pins, and wherein the raised pattern surface has a varying width profile, in the cross-machine direction, around the circumference of the anvil roll;
bonding at least the first and second supporting sheets together by transferring ultrasonic energy to the supporting sheets by means of the ultrasonic horn and the anvil,
wherein at a first circumferential position, a first part of the pattern surface, having a first width, interacts with the ultrasonic horn to provide a maximum bonding density, Wmax, and
wherein at a second circumferential position, a second part of the pattern surface, having a second width, interacts with the ultrasonic horn to provide a minimum bonding density, Wmin,
wherein the first and second pattern surfaces are configured so that the ratio of ultrasonic energy transfer at the first position to the ultrasonic energy transfer at the second position is from 1.1:1 to 10:1, preferably from 1.5:1 to 3:1.

In a preferred embodiment of the invention the ratio of the surface area of the pins at the first position to the surface area of the pins at the second position from 1.1:1 to 10:1, preferably from 1.5:1 to 3:1.

### Brief Description of the Drawings

Fig 1 is a schematic representation of an ultrasonic bonding unit suitable for the present invention.
Fig. 2 is a side view of an apparatus of the present invention, or used in the method of the invention.
Fig. 3 is a partial perspective view of an apparatus of the present invention, or used in a method of the invention.
Fig. 4 is a partial perspective view of an alternative apparatus of the present invention, or used in an alternative method of the invention.
Fig. 5A is a schematic representation of a bonding pattern made according to the apparatus or method of the present invention.
Fig. 5B is a is a schematic representation of an alternative bonding pattern made according to the apparatus or method of the present invention.

### Detailed Description of the Invention

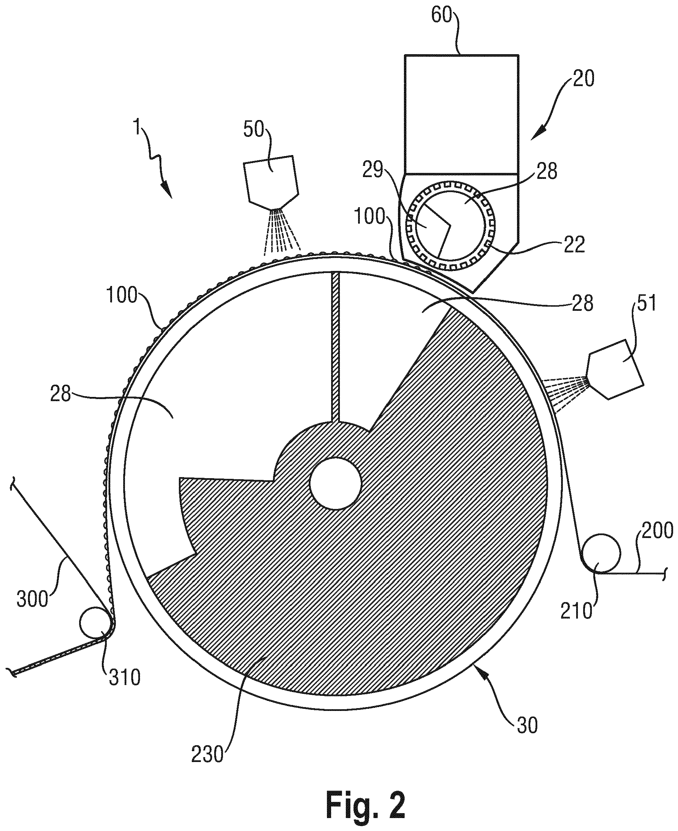

One embodiment of the invention is described below with reference to a method and apparatus applied to the manufacture of absorbent cores for disposable hygiene articles as shown in Figures 2, 3 and 4.

A pulsed stream of particles in a carrier means, such as air, as described in WO2003/101622, published on December 11th 2003.

In modern manufacturing processes, absorbent cores are formed continuously by depositing absorbent material, such as cellulose pulp, absorbent gelling material, or mixtures thereof onto a nonwoven substrate.

Disposable hygiene articles refers to a device that absorbs and contains body exudates, and, more specifically, refers to devices that are placed against or in proximity to the body of the wearer to absorb and contain the various exudates discharged from the body. Disposable hygiene articles may include adult and infant diapers, including pants, such as infant training pants and adult incontinence undergarments, and feminine hygiene products, such as sanitary napkins and panty-liners and adult incontinent pads, and breast pads, care mats, bibs, wound dressing products, and the like. As used herein, the term "body fluids" or "body exudates" includes, but is not limited to, urine, blood, vaginal discharges, breast milk, sweat and fecal matter.

Absorbent gelling material refers to a material or mixture of materials that can absorb and retain bodily fluids; it typically includes or consists of "superabsorbent polymer material". "Superabsorbent polymer material" (also known as "absorbent gelling material," or "AGM," or "superabsorbent,") refer to polymeric material that can absorb at least 10 times (and typically at least 15 times, or at least 20 times) their weight of an aqueous 0.9% saline solution as measured using the Centrifuge Retention Capacity test (Edana 441.2-02)., i.e. having a CRC of at least 10 g/g, and typically at least 20 g/g or at least 30 g/g.

In one exemplary process, particles of absorbent gelling material are deposited onto a nonwoven substrate in a controlled pattern by a process which is analagous to a gravure printing process.

For example, absorbent gelling material may be fed onto one or more supporting sheet by any suitable means including gravimetric feeding or by using a print roll. For example absorbent gelling material may be fed to a reservoir, the reservoir holds absorbent gelling material in a plurality of holes or cavities. The reservoirs may contain one or more channels between the absorbent regions, the channels being substantially free of absorbent material.

In a preferred embodiment, shown in Figure 2, the reservoirs 22 are disposed around the outer surface of print rolls. The absorbent gelling material 100 is delivered to a supporting sheet 200 by a print roll placed adjacent and in close proximity to the first moving endless surface, for example substantially above the surface. The absorbent gelling material 100 may be deposited substantially continuously. The point or area where the absorbent gelling material 100 leaves the print roll and transfers to the first moving endless surface is herein referred to as the depositing point or area; and in this point or area a raised strip 21, e.g. each raised strip, mates with a mating strip 31, e.g. without direct contact.

The supporting sheet(s) 200, 210 is/are preferably sheets of nonwoven material. Absorbent gelling material 100 is deposited onto at least one, and preferably both, of the supporting sheets from the print roll, except in the regions corresponding to the raised strips 21 and the mating strips 31.

Adhesives, such as hot melt adhesive, may be applied to the supporting sheet(s) before the absorbent gelling material is transferred to the supporting sheet at the depositing point and/or adhesive may be applied to the structure comprising the supporting sheet(s) and the absorbent gelling material 100 after the deposition point. The adhesive(s) may affix the absorbent gelling material 100 in place thereby maintaining the shape and bulk density of the absorbent area of the absorbent core which is formed by the absorbent gelling material 100.

An absorbent core may be formed by combining two supporting sheets 200, 210 each of which has been formed as described above so that two layers of absorbent gelling material 100 are sandwiched between outer supporting sheets 200, 210. Alternatively one supporting sheet which has been formed as described above may be combined with a separate sheet, e.g. a sheet of nonwoven material so that one layer of absorbent gelling material is sandwiched between outer sheets. Alternatively one supporting sheet may be folded so that absorbent gelling material is sandwiched between outer sheets.

Such a process is described in EP 1 621 166, published on February 1st 2006 and in WO2012/170798, published on December 13th 2012, both incorporated herein by reference.

Generally it is desirable to consolidate an absorbent core formed either by the process described above, or by another process, by sealing at least around the ends and sides of the absorbent core to minimise or prevent the escape of absorbent gelling material from within the core. Moreover sealing within the perimeter of the absorbent core, e.g. in the regions corresponding to the raised strips and the mating strips in the process described above, may provide regions which are free of absorbent gelling material. For example these regions may provide channels within the structure of the absorbent core.

It may be desirable to apply ultrasonic bonding to at least some of the end regions, side regions, channel regions or other regions of the absorbent core in order to maintain the profile and structural integrity of the absorbent core.

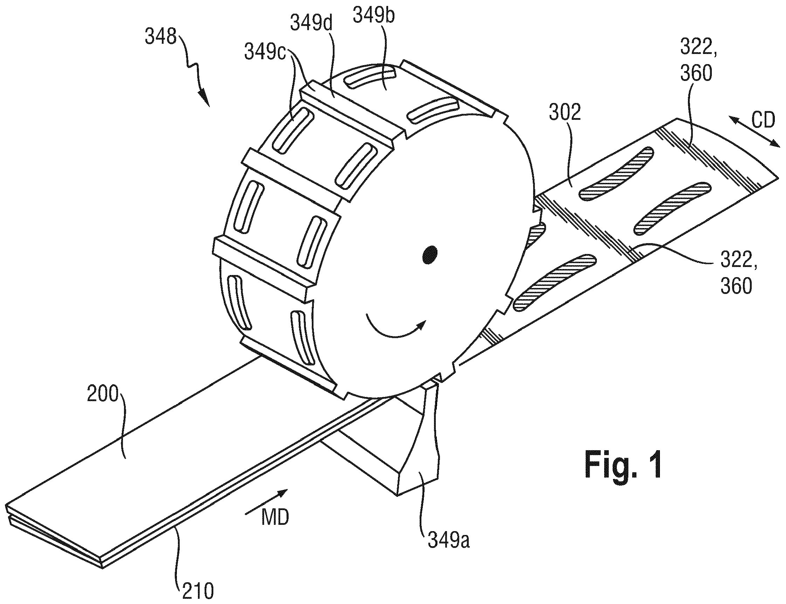

The apparatus and method of the present invention employ an ultrasonic bonding device such as schematically shown in Figure 1. The ultrasonic bonding device 348 includes a horn 349a which is configured to impart ultrasonic energy to first and second supporting sheets 200, 210 on a rotating anvil 349b. In turn, the anvil 349b includes a plurality of pattern elements 349c protruding radially outward from the anvil 349b, wherein each pattern element includes a pattern surface 349d. It is to be appreciated that the number, size, and shape of some or all the pattern surfaces and/or pattern elements varies around the circumference of the anvil 349b. In some configurations, the pattern elements 349c and/or pattern surfaces 349d may have a perimeter that defines circular, square, rectangular, elliptical, and various types of other shapes. In some configurations, the anvil 349b may include a pattern element 349c with a pattern surface 349d that defines a continuous crossing line pattern and/or various other shapes, such as disclosed in U.S. Patent No. 9,265,672, which is incorporated by reference herein. Patterns with different perimeter defining circle, ellipse, rectangle or various other shapes can be different for different regions along the machine direction. Different regions of bonding density are defined by Wmax and Wmin where ultrasonic horn and anvil interact to provide bonding. The bonding density is defined below. It is to be appreciated that the pattern surface 349d, such as discussed above may also include regions defined by relatively high and relatively low elevations. Thus, such pattern surfaces may create bonds 322 having varying thicknesses across the bond region 360. It is to be appreciated that the choice of pattern surface shape may enable the creation of unique textures and patterns where the location and size of the bonding sites impact local buckling resistance of a nonwoven laminate and may create desired homogeneous textures.

With continued reference to Figure 1, the ultrasonic bonding device applies energy to the horn 349a to create resonance of the horn at frequencies and amplitudes so the horn vibrates rapidly in a direction generally perpendicular to the first and second substrates 200, 210 being advanced past the horn 349a on the anvil 349b. Vibration of the horn 349a generates heat to melt and bond the supporting sheets 200, 210 together in areas supported by the pattern elements 349c on the anvil 349b. Thus, the bonds 322 and/or bond regions 360 have shapes that correspond with and may mirror shapes of the pattern surfaces 349d. As shown in Figure 1, the pattern surface 349d may extend contiguously across the first supporting sheet 200, and the second supporting sheet 210. It is to be appreciated that aspects of the ultrasonic bonding devices may be configured in various ways, such as for example linear or rotary type configurations, and such as disclosed for example in U.S. Patent Nos. 3,113,225; 3,562,041; 3,733,238; 5,110,403; 6,036,796; 6,508,641; and 6,645,330.

In some configurations, the ultrasonic bonding device may be configured as a linear oscillating type sonotrode, such as for example, available from Herrmann Ultrasonic, Inc. Although the bond applicator 348 is shown in Figure 1 as a separate device that is positioned downstream of the metering device 1, it is to be appreciated the metering device and the bond applicator 348 may be integrated into a single unit. As such, the first supporting sheet 200, second supporting sheet 210 may be combined and bonded together at the bond applicator 348 to form the absorbent core.

The bonding density is defined herein as the bonded area per centimeter, in the machine direction, of the pattern surface. For the purpose of establishing maximum bonding density, Wmax, and minimum bonding density, Wmin, it is necessary to consider regions which fully encompass the bonding pattern, i.e. excluding regions which have no bonding pattern. The bonded area defined herein may refer to the bonded area of the absorbent core, or the bonded area may refer to the corresponding area of the pattern surface, comprising a plurality of pins, of the anvil roll.

The magnitude of ultrasonic energy transfer may be measured by using the signal which comes from the ultrasonic generator. Accordingly ratios of ultrasonic energy transfer may be determined.

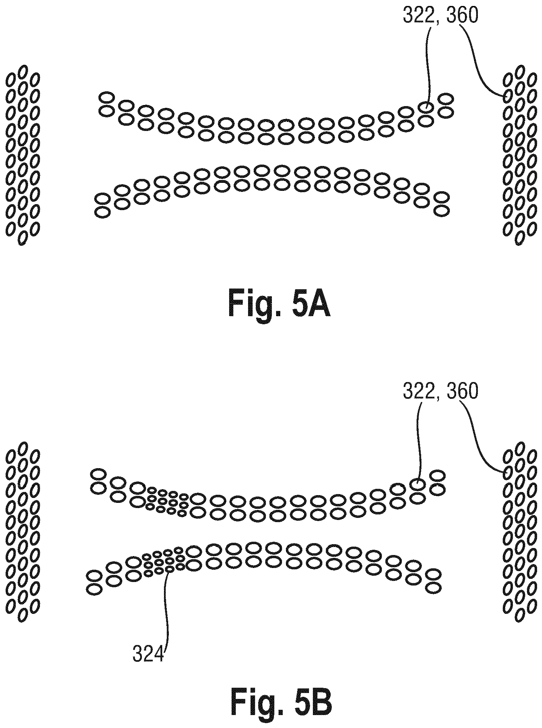

Some of the examples for pin pattern as described in the application can have rounded shapes in Wmax or/and Wmin regions. The diameter of the pins shape can be varied to differentiate the energy requirement. One possible example may consist of circular pins in the center of the product and elliptical pins in the end of the product, also known as end flap regions. Circular pins may have diameter below 2mm and elliptical pins may be 2.66mm long and 0.66mm wide. Elliptical pins in end flap regions can have width up to 2mm wide. Pins which have rounded shapes are preferred for the channel regions to avoid longitudinal shapes and avoid tears/slits along the channels with a diameter below 2mm to avoid holes bigger than 2mm in case the bond becomes a hole after process. For example, the channel region may have pins having dimensions of 2.66mm long and 0.6mm wide; the end flap region may have pins of 1.66mm long and 0.6mm wide, as illustrated in Figure 5A.

Figure 5B illustrates an alternative version of the pin pattern which has different patterns, 322, 324 within the channel region to create bond regions where the resilience is higher than in adjacent bond regions. In use the absorbent core absorbs liquid and swells. Therefore the absorbent core illustrated in Figure 5B releases some of the swelling constraint and creates folding lines in some part of the bond region 324. In such a pattern, different area or perimeter of pins in center of the product or in channels pins with smaller diameter can be added with pins of larger diameter.

Another method to optimize the bond strength is to preheat the nonwoven web in end flap area, for example by adding small amount of hot melt glue or by passing the substrate over hot drum. This method further optimizes the bond strength. Hot melt will reduce the ultrasonic energy requirement for melting thereby allowing to have an anvil with higher ratio of Wmax to Wmin. This glue can be added to whole nonwoven surface or part of the nonwoven surface such as in end flap regions. To reduce the glue or holt melt usage amount temperature of the hot melt can be varied.

A particularly preferred process for the manufacture and construction of absorbent cores is described in further detail below.

As shown in Figure 2, the absorbent material 100 is delivered to the supporting sheet 200 by a first moving endless surface placed adjacent and in close proximity to said second moving endless surface, for example substantially above said surface. The absorbent material 100 may be deposited substantially continuously. The point or area where the absorbent material 100 leaves the first moving endless surface and transfers to said second moving endless surface 30 is herein referred to as meeting point; and in this point or area a raised strip 21, e.g. each raised strip, mates with a mating strip 31, e.g. without direct contact.

A feeder 60 may deliver the absorbent material 100 to said first moving endless surface. Such as feeder 60 is capable of containing the absorbent material 100 and letting it flow to the supporting sheet 200 on said second moving endless surface, for example continuously. The feeder 60 may be a (e.g. stationary) hopper with a container portion, to hold the material, e.g. having a volume of at least 1000 cm³, and it may have a guiding portion, e.g. a pipe-shapes portion, that guides the material from the container portion to the first moving endless surface.

The first moving endless surface may be a rotating roll or drum, as for example shown in the Figures 2, 3 and 4. The radius of the first moving endless surface may depend on what absorbent structure is produced, e.g. what size, and for example how many structures are produced per cycle of the first moving endless surface, e.g. print roll or drum.

The first moving endless surface may have any suitable width, but for example a width (for example in CD, hence perpendicular to MD) corresponding (substantially) to the width of the absorbent structure to be produced; this for example be at least 40 mm, or at least 60 mm, or for example up to 400 mm, or up to 200 mm.

Said first moving endless surface may have one or more reservoirs with a certain volume for receiving said absorbent material 100 therein, and transporting it to and then depositing it on said supporting sheet 200 on a second moving endless surface.

Each reservoir 22 corresponds typically to an absorbent structure to be produced, as suitable for an absorbent article. The supporting sheet 200 may be a web material, so the method and apparatus 1 herein can thus serve to produce a web of such absorbent structures that are then subsequently separated into individual structures.

The reservoir 22 is at least partially air-permeable. It typically has an area that serves to receive said absorbent material, and this area is substantially in air-communication with a vacuum system, i.e. air permeable.

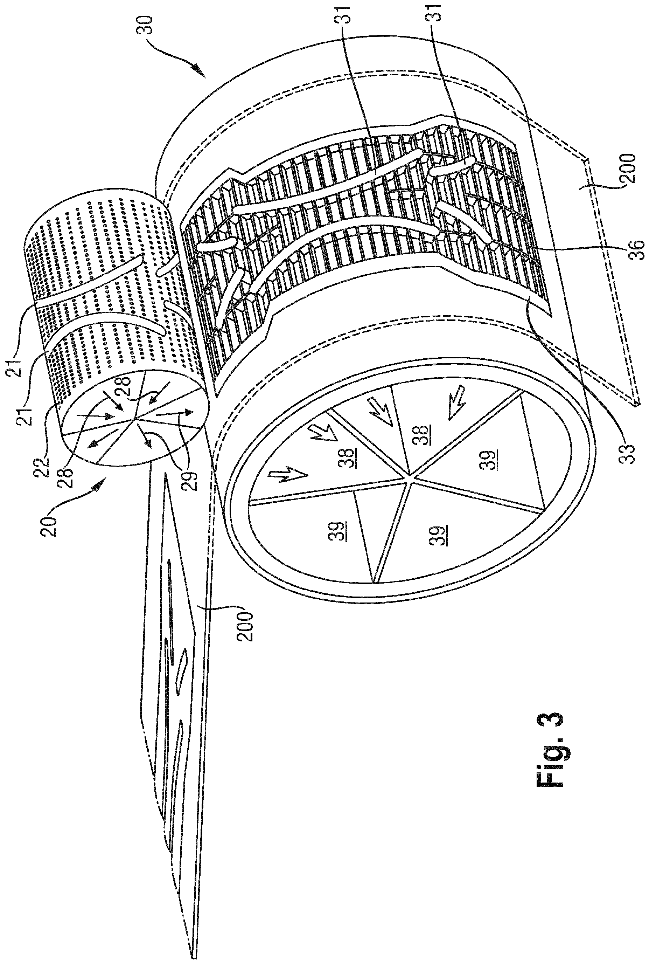

As for example shown in Figure 3, the reservoir 22 has one or more raised strips 21 (that have no void volume) and that, when the first moving endless surface moves (rotates) adjacent said second moving endless surface 30 with said supporting sheet 200 on said mating strips 31, said raised strips 21 mate substantially coincide (herein referred to as: mate) with said mating strips 31. Then, the absorbent material 100 is deposited selectively on the substrate material on the portions thereof that are not on said mating strips 31, to form an absorbent layer on a supporting sheet 200, having channels (strips) that are substantially free of absorbent material. A reservoir 22 has typically the same number of raised strips 21 as the number of mating strips 31 of the second moving endless surface.

Said strip(s) 21 may not be in air communication with a vacuum system that is in air communication with said first moving endless surface, i.e. the raised strips 21 may be air impermeable. They may have thereto have surfaces that have no apertures. The remaining area of the reservoir 22 that has void volume for receiving the absorbent material, thus excluding the raised strips 21, may be in air-communication with a vacuum system, e.g. having apertures in air-communication with a vacuum system.

The reservoir 22 has peripheral edges, and peripheral edge zones, including opposing longitudinal edges and edge zones, and a transverse front edge and front edge zone, and a transverse back edge and back edge zone. Each of said front and back edge zones, extending the complete transverse dimension, may for example have a longitudinal dimension of from about 5% to about 20%, or to 15%, or to 10% of the average longitudinal dimension of the reservoir. Each of said longitudinal edge zone may extend the length of the reservoir 22 and may have an average transverse dimension of for example from about 5% to about 20%, or typically to about 15% or to about 10% of the total average transverse dimension of the reservoir. In some embodiments herein, said raised strip(s) are not present in any of said edge zones.

The reservoir 22 may in addition, or alternatively, comprise a front region, back region and central region, therein between, as further described below. The central region may be for example the central 1/3 of the reservoir, extending the full transverse dimension. In some embodiment, the raised strips 21 are present only in the front region; alternatively, in some embodiments, the raised strips 21 are present in the central region only or at least; alternatively, in some embodiments, the raised strips 21 are present in the central region and front region and optionally in the back region. It may alternatively or in addition be preferred that one or more raised strips 21 are present in the central region and front region. To provide improved liquid transportation and more efficient absorbency by the whole absorbent structure, it may be preferred to have said raised strip(s) at least in the central region and preferably extending also at least in the front region.

In any such case, it may be preferred that none of the edge zones described above comprises such raised strips 21.

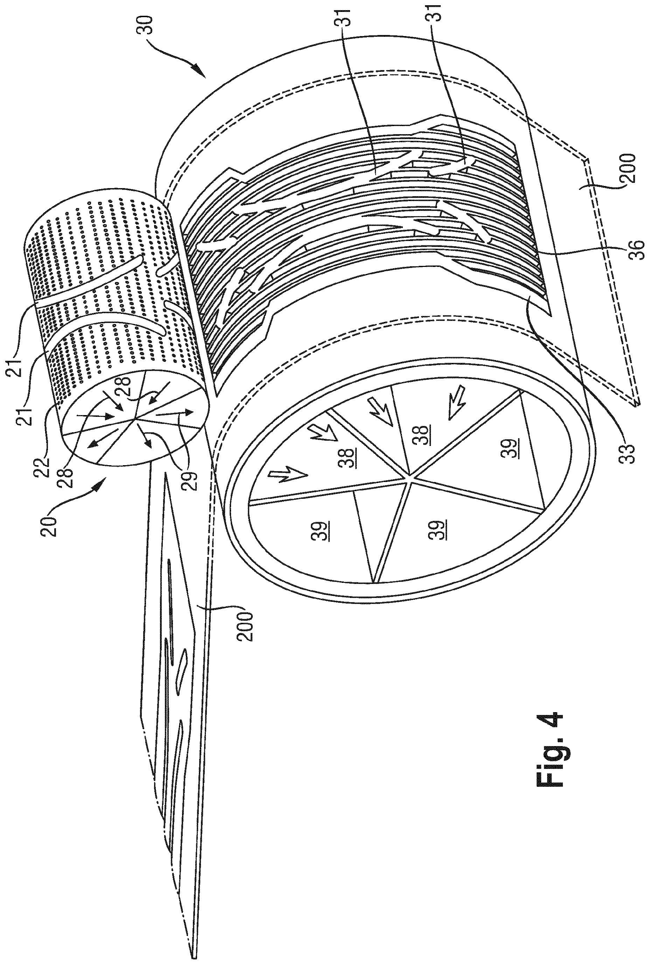

As for example shown in Figures 3 and 4, a raised strip 21 meets (and hence mates with) a mating strip 31 during the transfer of the absorbent material 100 herein, i.e. during absorbent material 100 transfer to said second moving endless surface, said raised strip 21 substantially overlaps said mating strip. This typically applies to each mating raised strip 21 and mating strip. In other words, each raised strip 21 typically has a corresponding mating strip. It should be understood that the raised strip 21 and mating strip 31 do not directly touch during the absorbent material 100 transfer (the supporting sheet 200 material is in between them). The supporting sheet 200 may or may not touch the raised strip(s).

The following is described for a raised strip, in relation to its corresponding mating strip, but may be, applicable to each raised strip.

A raised strip 21 and the corresponding mating strip 31 may have the same size and shaped and surface area, and be hence completely mating. It may be that a raised strip 21 has a surface area that is slightly more than the surface area of a corresponding mating strip. Hereby, the mating strip 31 and the corresponding raised strip 21 then typically still have substantially the same overall shape.

### Absorbent material

The absorbent material 100 herein is preferably a flowable material (in the dry state), such as a particulate material; it may be any material in particulate form, which includes particles, flakes, fibers, spheres, agglomerated particles and other forms known in the art.

The absorbent material 100 comprises superabsorbent polymer material (e.g. particles), optionally combined with cellulosic material (including for example cellulose, comminuted wood pulp in the form of fibers).

In some embodiments herein, the absorbent material, e.g. the particulate absorbent material, comprises at least, or consists essentially of or consists of, (particulate) superabsorbent polymer material, herein referred to as SAP, and also known as particulate absorbent gelling material, AGM. The particulate SAP herein may have a high sorption capacity, e.g. having a CRC of for example at least 20 g/g, or at 30 g/g. Upper limits may for example be up to 150 g/g, or up to 100 g/g.

In some embodiments herein, the absorbent material 100 comprising or consisting of particles of polyacrylic acids/ polyacrylate polymers, for example having a neutralization degree of from 60% to 90%, or about 75%, having for example sodium counter ions, as known in the art; they may be surface crosslinked and/ or internally crosslinked polyacrylic acid/ polyacrylate polymers.
In some embodiments herein, the absorbent material 100 is in the form of particles with, a mass medium particle size up to 2 mm, or between 50 microns and 2 mm or to 1 mm, or preferably from 100 or 200 or 300 or 400 or 500µm, or to 1000 or to 800 or to 700 µm; as can for example be measured by the method set out in for example EP-A-0 691 133.

### Supporting sheet

The absorbent structure producible with the apparatus 1 and method of the invention comprises a supporting sheet 200, to receive the absorbent material. This supporting sheet 200 may be any individual sheet or web sheet material, in particular paper, films, wovens or nonwovens, or laminate of any of these.

In some embodiments herein, the supporting sheet 200 is a nonwoven, e.g. a nonwoven web, such as a carded nonwoven, spunbond nonwoven or meltblown nonwoven, and including nonwoven laminates of any of these.

The fibers may be of natural or man-made origin and may be staple or continuous filaments or be formed in situ. Commercially available fibers have diameters ranging typically from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). The fibers may be bicomponent fibers, for example having a sheet-core arrangement, e.g. with different polymers forming the sheet and the core. Nonwoven fabrics can be formed by many processes such as meltblowing, spunbonding, solvent spinning, electrospinning, and carding. The basis weight of nonwoven fabrics is usually expressed in grams per square meter (gsm).

The nonwoven herein may be made of hydrophilic fibers; "Hydrophilic" describes fibers or surfaces of fibers, which are wettable by aqueous fluids (e.g. aqueous body fluids) deposited on these fibers. Hydrophilicity and wettability are typically defined in terms of contact angle and the strike through time of the fluids, for example through a nonwoven fabric. This is discussed in detail in the American Chemical Society publication entitled "Contact angle, wettability and adhesion", edited by Robert F. Gould (Copyright 1964). A fiber or surface of a fiber is said to be wetted by a fluid (i.e. hydrophilic) when either the contact angle between the fluid and the fiber, or its surface, is less than 90°, or when the fluid tends to spread spontaneously across the surface of the fiber, both conditions are normally co-existing. Conversely, a fiber or surface of the fiber is considered to be hydrophobic if the contact angle is greater than 90° and the fluid does not spread spontaneously across the surface of the fiber. The supporting sheet 200 herein may be air-permeable. Films useful herein may therefore comprise micro pores. Nonwovens herein may for example be air permeable. The supporting sheet 200 may have for example an air-permeability of from 40 or from 50, to 300 or to 200 m³/ (m²x min), as determined by EDANA method 140-1-99 (125 Pa, 38.3 cm²). The supporting sheet 200 may alternatively have a lower air-permeability, e.g. being non-air-permeable, to for example be better detained on a moving surface comprising vacuum.

In preferred executions, the supporting sheet 200 is a nonwoven laminate material, a nonwoven laminate web, for example of the SMS or SMMS type.

In order to form easily said undulations, the supporting sheet 200 may have a basis weight that is less than 60 gsm, or for example than 50 gsm, for example from 5 gsm to 40 gsm, or to 30 gsm.

The supporting sheet 200 may have a transverse-extensibility or a longitudinal-extensibility, for example of more the 20 %, or for example more than 100 %, but for example not more than 200 %.

In one of the embodiment herein, the supporting sheet 200 has a transverse dimension that is more than the transverse dimension of the part of the receptacle 33, e.g. at least 10%, or for example at 20% or at least 30%, and for example up to about 120%.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An apparatus comprising an ultrasonic horn (349a) and an anvil roll (349b),
wherein the anvil roll (349b) comprises a plurality of raised pattern elements (349c) around the circumference of the anvil roll (349b),
wherein the pattern elements (349c) comprising a pattern surface (349d) comprising a plurality of pins, the raised pattern surface (349c) has a varying width profile, in the cross-machine direction, around the circumference of the anvil roll (349b),
wherein at a first circumferential position, a first part of the pattern surface (349d), having a first width, interacts with the ultrasonic horn (349a) to provide a maximum bonding density, Wmax, and
wherein at a second circumferential position, a second part of the pattern surface (349d), having a second width, interacts with the ultrasonic horn (349a) to provide a minimum bonding density, Wmin,
wherein the first and second parts of the pattern surface (349d) are configured so that the ratio of ultrasonic energy transfer at the first position to the ultrasonic energy transfer at the second position is from 1.1:1 to 10:1.

2. The apparatus according to Claim 1 wherein the first and second parts of the pattern surfaces (349d) are configured so that the ratio of ultrasonic energy transfer at the first position to the ultrasonic energy transfer at the second position is from 1.5:1 to 3:1.

3. The apparatus according to Claim 1 wherein the first and second parts of the pattern surfaces (349d) are configured so that the ratio of the surface area of the pins at the first position to the surface area of the pins at the second position is from 1.1:1 to 10:1.

4. A method comprising the steps of:
advancing a composite of at least first and second supporting sheet (200, 210) through a bonding region, wherein the bonding region comprises an ultrasonic horn (349a) and an anvil roll (349b), the anvil roll (349b) comprising a plurality of raised pattern elements (349c) around the circumference of the anvil roll (349b), the pattern elements (349c) comprising a pattern surface (349d) comprising a plurality of pins, and wherein the raised pattern surface (349c) has a varying width profile, in the cross-machine direction, around the circumference of the anvil roll (349b);
bonding at least the first and second supporting sheets (200, 210) together by transferring ultrasonic energy to the supporting sheets (200, 210) by means of the ultrasonic horn (349a) and the anvil (349b),
wherein at a first circumferential position, a first part of the pattern surface (349d), having a first width, interacts with the ultrasonic horn (349a) to provide a maximum bonding density, Wmax, and
wherein at a second circumferential position, a second part of the pattern surface (349d), having a second width, interacts with the ultrasonic horn (349a) to provide a minimum bonding density, Wmin,
wherein the first and second pattern surfaces are configured so that the ratio of ultrasonic energy transfer at the first position to the ultrasonic energy transfer at the second position is from 1.1:1 to 10:1.

5. The method according to Claim 4 wherein the first and second parts of the pattern surfaces (349d) are configured so that the ratio of ultrasonic energy transfer at the first position to the ultrasonic energy transfer at the second position is from 1.5:1 to 3:1.

6. The method according to Claim 4 wherein the first and second parts of the pattern surfaces (349d) are configured so that the ratio of the surface area of the pins at the first position to the surface area of the pins at the second position is from 1.1:1 to 10:1.

7. The method according to any of Claims 4 to 6 wherein at least one of the first or second supporting sheets (200, 210) is heated by the ultrasonic horn (349a) and the anvil (349b), and wherein an additional source of heat is provided in at least at the second circumferential position.

8. The method according to Claim 7 wherein the additional source of heat is provided by the application of a hot melt glue at the second circumferential position.
